# DEMANDE DE BREVET EUROPEEN

(11) **EP 1 118 321 A1**
(43) Date de publication de la demande: **25.07.2001**
(21) Numéro de dépôt: 00100721.0
(22) Date de dépôt: 14.01.2000
(51) Int. Cl.: A61K 9/20

(54) **Compositions pharmaceutiques solides pour la libération contrôlée de substances actives**

(71) Demandeur: UCB, S.A., 1070 Bruxelles (BE)
(72) Inventeur: Fanara, Domenico, 4520 Wanze (BE); Deleers, Michel, 1630 Linkebeek (BE); Guichaux, Anthony, 1950 Kraainem (BE); Berwaer, Monique, 6120 Ham-Sur-Heure-Nalinnes (BE)
(74) Mandataire: Lechien, Monique

(57) **Abrégé**

La présente invention concerne des compositions pharmaceutiques solides administrables par voie orale, permettant la libération contrôlée d'au moins une substance active. L'invention concerne aussi des procédés de préparation de ces compositions, et les utilisations de ces compositions pharmaceutiques.

## Description

La présente invention concerne des compositions pharmaceutiques solides administrables par voie orale permettant la libération contrôlée de substances pharmaceutiquement actives ainsi que des méthodes de préparation de ces compositions pharmaceutiques.

Un des buts recherchés actuellement dans le développement de compositions pharmaceutiques solides administrables par voie orale est de contrôler la libération des substances pharmaceutiquement actives de manière à ce qu'elles puissent être administrées en peu de prises journalières, idéalement en une seule prise journalière.

Le contrôle de la libération de substances actives lors de l'administration par voie orale peut se faire au moyen de compositions pharmaceutiques de type matriciel. Selon les excipients utilisés, on distingue trois types de matrices : les matrices inertes, hydrophiles et lipophiles. Par association d'excipients de ces différents types de matrices, on peut aussi créer des matrices mixtes.

Les matrices inertes comprennent des excipients appartenant essentiellement à la classe des polymères thermoplastiques. Ils sont généralement inertes vis-à-vis des tissus biologiques, des autres excipients dans la formulation et de la substance active. Ils sont insolubles et non-digestibles dans les fluides du tractus gastro-intestinal. Parmi ceux-ci, on peut citer le chlorure de polyvinyle, le polyéthylène, les copolymères d'acétate et de chlorure de vinyle, les polyméthylméthacrylates, les polyamides, les silicones, l'éthylcellulose, le polystyrène ... Ils s'utilisent généralement à une concentration allant de 20 à 95%.

Les matrices hydrophiles comprennent des excipients gélifiants se répartissant en trois classes : les dérivés cellulosiques (hydroxypropylméthylcellulose, hydroxyéthylcellulose, hydroxypropylcellulose, méthylcellulose ...), les polysaccharides non cellulosiques (galactomannes, gomme guar, gomme caroube, gomme arabique, gomme sterculia, agar agar, alginates ...) et les polymères de l'acide acrylique (produits vendus sous la marque Carbopols 934P et 974P ...). Ils s'utilisent généralement à une concentration de 20 à 70%.

Les matrices lipidiques comprennent des excipients gras de quatre types : les glycérides (mono- di- ou triglycérides : stéarine, palmitine, laurine, myristine, huiles de ricin ou de coton hydrogénées, produit vendu sous la marque Précirol ...), les acides et les alcools gras (acides stéarique, palmitique, laurique; alcools stéarylique, cétylique, cétostéarylique ...), les esters d'acides gras (monostéarates de propylène glycol et de saccharose, distéarate de saccharose ...) et les cires (cire blanche, cire de cachalot ...). Ils s'utilisent généralement à une concentration de 10 à 50%.

La présence d'excipients de type matriciel dans des compositions pharmaceutiques permet dans bien des cas de ralentir la libération des substances actives par emprisonnement. Toutefois, ces excipients de type matriciel ne permettent pas toujours de ralentir suffisamment la libération de la substance active ou d'obtenir les profils de libération idéaux souhaités.

Par exemple, lorsque la composition pharmaceutique de type matriciel contient une substance qui doit impérativement être libérée dans l'estomac, la libération de la substance active pendant des durées suffisamment longues dépend non seulement du type d'excipients utilisés dans la composition, mais aussi du temps de résidence de la composition pharmaceutique dans l'estomac. C'est pourquoi plusieurs documents mentionnent l'utilisation de comprimés matriciels flottants.

En particulier, le brevet EP 205336 décrit des compositions pharmaceutiques pour la libération contrôlée de substances actives comprenant une matrice mixte obtenue à partir d'un mélange d'éthers de cellulose et d'un acide polyacrylique, de l'un de ses dérivés ou de leurs sels pharmaceutiquement acceptables, et comprenant en outre de 10 à 50% en poids, par rapport au poids total d'excipients matriciels, d'agent moussant effervescent. L'agent moussant effervescent permet de faire flotter la composition pharmaceutique dans le liquide gastrique, augmentant par là-même le temps de résidence dans l'estomac. L'agent moussant effervescent est un bicarbonate de métal alcalin ou alcalino-terreux utilisé de préférence en combinaison avec un acide organique.

Toutefois, la flottaison dans le liquide gastrique ne permet pas de résoudre d'autres problèmes observés dans le contexte du contrôle de la libération de substances actives à partir de compositions pharmaceutiques matricielles.

En effet, les quantités d'excipient matriciel nécessaires à une libération prolongée adéquate du principe actif peuvent se révéler trop importantes et rendre impossible ou trop onéreuse la réalisation de la forme pharmaceutique.

D'autre part, la libération de certaines substances actives dépend fortement du pH. Par exemple, certaines substances actives ne sont pas du tout libérées dans l'estomac, mais dans d'autres zones du tractus gastro-intestinal. En outre, pour une même zone du tractus gastro-intestinal, le profil de libération sera différent selon que l'administration de la composition a lieu simultanément ou non avec la prise d'aliments. Pour les substances actives dont la libération dépend du pH ambiant, il est donc souhaitable de trouver de nouvelles compositions matricielles permettant de régulariser la vitesse de libération de manière à ce que la substance active puisse être libérée à la même vitesse, quel que soit le pH du milieu.

Enfin, il est très courant que le profil de libération d'un principe actif à partir d'une forme matricielle soit irrégulier au cours du temps, c'est-à-dire que la cinétique de libération ne soit pas d'ordre zéro mais soit fonction de la racine carrée du temps. Une cinétique de libération d'ordre zéro correspond à une libération régulière et constante au cours du temps et est très recherchée pour garantir un effet thérapeutique régulier et de longue durée.

Parallèlement, il devient thérapeutiquement de plus en plus intéressant de pouvoir administrer simultanément par voie orale une substance active libérée immédiatement après administration, et la même ou une deuxième substance active libérée de manière progressive et régulière après administration. Dans le cas où la même substance active est simultanément administrée en libération immédiate et en libération prolongée, cela permet de libérer rapidement une dose suffisante de substance active pour déclencher l'effet désiré et de maintenir cet effet par une libération progressive et prolongée de la même substance active. Dans le cas où une substance active est libérée immédiatement et une autre substance active est libérée de manière prolongée, cela permet d'obtenir des effets thérapeutiques combinés au moyen de deux substances actives ayant des profils pharmacocinétiques très différents.

Dans la demande de brevet international (PCT/BE98/00033), déposée au nom d'UCB, S.A., des nouvelles compositions pharmaceutiques administrables par voie orale, permettant la libération contrôlée de substances pharmaceutiquement actives de manière telle qu'un effet thérapeutique satisfaisant est observé pendant des durées assez longues, par exemple en une, voire deux prises journalières seulement, sont décrites.

Cette demande de brevet concerne des compositions pharmaceutiques administrables par voie orale, permettant la libération contrôlée d'au moins une substance active, comprenant
a) ladite au moins une substance active,
b) entre 5 et 60% en poids, par rapport au poids total de la composition, d'au moins un excipient, sélectionné parmi les matrices inertes, les matrices hydrophiles, les matrices lipidiques, les mélanges de matrices inertes et de matrices lipidiques, les mélanges de matrices hydrophiles et de matrices lipidiques, les mélanges de matrices hydrophiles et de matrices inertes, à l'exception des mélanges comprenant un acide polyacrylique et au moins une matrice hydrophile de type cellulosique;
c) entre 5 et 50% en poids, par rapport au poids total de la composition d'au moins un agent alcalinisant soluble dans une phase aqueuse dans des conditions de pH physiologique, sélectionné parmi les hydroxydes, les carbonates, les bicarbonates et les phosphates de métaux alcalins ou alcalino-terreux, le borate de sodium ainsi que les sels basiques d'acides organiques.

Poursuivant nos recherches dans ce domaine, nous avons développé de nouveaux types de compositions solides aux avantages inattendus. Nous venons en effet de découvrir de manière surprenante de nouvelles compositions pharmaceutiques solides à libération contrôlée administrables par voie orale, permettant d'obtenir l'indépendance de la libération de la substance active vis-à-vis du pH environnant. Ainsi, in vivo, cette composition est de nature à assurer une libération régulière et totale de la substance active tout au long du tractus gastro-intestinal. Elle permet d'obtenir une linéarité de la cinétique de libération jamais atteinte jusqu'à présent dans des conditions de pH variables.

Cette nouvelle invention concerne donc des compositions pharmaceutiques solides administrables par voie orale, permettant la libération contrôlée d'au moins une substance active, comprenant
a) la dite au moins une substance active;
b) entre 5 et 95 % en poids, par rapport au poids total de la composition, d'au moins un excipient matriciel, sélectionné parmi les matrices inertes, les matrices hydrophiles, les matrices lipidiques, les mélanges de matrices inertes et de matrices lipidiques, les mélanges de matrices hydrophiles et de matrices lipidiques, les mélanges de matrices hydrophiles et de matrices inertes;
c) entre 2 et 50 % en poids, par rapport au poids total de la composition, d'au moins un polymère entérosoluble ; et
d) entre 0,5 et 50 % en poids, par rapport au poids total de la composition, d'au moins un agent alcalinisant soluble dans une phase aqueuse dans des conditions de pH physiologique.

Nous entendons par polymères entérosolubles (ou polymères entériques) des polymères portant au moins un groupement acide, tels que par exemple, des groupements carboxyliques. Ces groupements acides permettent de rendre les polymères entérosolubles insolubles en milieu acide stomacal et ainsi de ralentir la libération des substances actives. Ces mêmes groupements acides forment un sel soluble au pH du milieu intestinal d'où la solubilité du polymère dans l'intestin. C'est pourquoi, à pH alcalin, le polymère entérosoluble va se dissoudre et provoquer des cavités par érosion. Le réseau creux ainsi obtenu dans la composition pharmaceutique solide, va faciliter la libération des substances actives.

Les compositions pharmaceutiques solides selon la présente invention comprennent des polymères entérosolubles qui peuvent être choisis parmi les polymères cellulosiques ou les polymères acryliques.

Des exemples de polymères entérosolubles cellulosiques utilisables selon la présente invention sont l'hydroxypropylméthylcellulose acétate succinate (composés vendus sous la marque Aqoat par Shin-Etsu ), l'acétylphtalate de cellulose, le phtalate d'hydroxypropylméthylcellulose ou la cellulose acétate trimellitate (Aimley Wade and Paul J. Weller 'Handbook of Pharmaceutical Excipents' American Pharmaceutical Association (1994) p 233-237).

Des exemples de polymères entérosolubles acryliques utilisables selon la présente invention sont les copolymères de l'acide méthacrylique, tels que les composés vendus sous la marque Eudragit par Röhm Pharma GmbH (Aimley Wade and Paul J. Weller 'Handbook of Pharmaceutical Excipents' American Pharmaceutical Association (1994) p 362-366).

La teneur en polymères entérosolubles selon la présente invention est comprise entre 2 et 50 % en poids, par rapport au poids total de la composition. Habituellement, elle est comprise entre 2 et 30 % et, préférentiellement, entre 5 et 15 % en poids, par rapport au poids total de la composition.

L'agent alcalinisant utilisable selon la présente invention doit être soluble dans une phase aqueuse dans des conditions de pH physiologique pour produire l'effet désiré. L'agent alcalinisant peut être choisi parmi les hydroxydes, les carbonates, les bicarbonates, les phosphates de métaux alcalins ou alcalino-terreux, le borate de sodium ainsi que des sels basiques d'acides organiques (exemple: citrate de sodium). Par contre, les sels non solubles dans l'eau dans les conditions de pH physiologique tels que stéarate de magnésium, ou phosphate dibasique de calcium, ne conviennent pas selon la présente invention.

La quantité d'agent alcalinisant présente dans les compositions pharmaceutiques selon la présente invention est habituellement de 0,5 à 50 % en poids par rapport au poids total de la composition, et préférentiellement entre 5 à 50 %.

Les compositions pharmaceutiques solides selon la présente invention comprennent des excipients matriciels choisis parmi les matrices inertes, hydrophiles et lipidiques.

Des exemples de matrices inertes utilisables selon la présente invention sont: le chlorure de polyvinyle, le polyéthylène, les copolymères d'acétate et de chlorure de vinyle, les polyméthylméthacrylates, les polyamides, les silicones, l'éthylcellulose, le polystyrène.

Des exemples de matrices hydrophiles utilisables selon la présente invention sont: les dérivés cellulosiques (hydroxypropylméthylcellulose, hydroxyéthylcellulose, hydroxypropylcellulose, méthylcellulose ...), les polysaccharides non cellulosiques (galactomannanes, gomme guar, gomme caroube, gomme arabique, gomme sterculia, agar agar, alginates ...) et les polymères de l'acide acrylique (carbopols 934P et 974P ...fabriqués par BF Goodrich Company). Les matrices hydrophiles utilisées préférentiellement selon la présente invention sont les hydroxypropylméthylcelluloses.

Des exemples de matrices lipidiques utilisables selon la présente invention sont: les glycérides (mono- di- ou triglycérides: stéarine, palmitine, laurine, myristine, huiles de ricin ou de coton hydrogénées, précirol ...), les acides et les alcools gras (acides stéarique, palmitique, laurique; alcools stéarylique, cétylique, cétostéarylique ...), les esters d'acides gras (monostéarates de propylène glycol et de saccharose, distéarate de saccharose ...) et les cires (cire blanche, cire de cachalot ...).

Les excipients matriciels peuvent également se trouver sous la forme de mélange.

En ce qui concerne les substances actives qui peuvent être présentes dans les compositions selon la présente invention, elles peuvent être de nature très variée. Elles peuvent être choisies, par exemple, parmi les vasoconstricteurs, les antihistaminiques, les analgésiques, les antitussifs, les antiépileptiques, neuroleptiques, anxyolitiques, normothymiques, antidépresseurs, antimigraineux, les composés traitant l'accès manique (composés antimaniques), les troubles bipolaires, la diskinesie, la myoclonie. Des exemples non limitatifs de telles substances actives sont la pseudoéphédrine, l'éphédrine, la phényléphrine, la phénylpropanolamine, le trapidil, l'hydrocodone, la cétirizine, l'éflétirizine, l'hydroxyzine, la méclozine, la buclizine, la pentoxyvérine, la codéine, la morphine, la théophylline, le lévétiracetam, le piracétam, le valpromide, le valproate de sodium, la gabapentine, la carbamazépine, leurs isomères optiques ou leurs sels pharmaceutiquement acceptables.

Cette composition est également particulièrement bien adaptée pour libérer des substances actives ayant des caractéristiques basiques.

Quant à la dose de substance active utilisée, elle dépend de la dose efficace et peut donc varier dans des limites très larges dépendant de ladite substance active.

Outre les composants précités, les compositions pharmaceutiques solides selon la présente invention peuvent également contenir d'autres excipients tels que des diluants (produit de marque Emcompress, lactose ...), des liants (produit de marque Avicel, amidons, polyvinylpyrrolidone ...), des désintégrants (amidons et amidons modifiés, dérivés cellulosiques, dérivés alginiques, pectines ...), des lubrifiants et agents d'écoulement (talc, stéarate de magnésium, silice colloïdale ...), des agents de masquage de goût (α-cyclodextrine, β-cyclodextrine, γ-cyclodextrine et leurs dérivés ), des arômes ou des colorants ainsi que des agents de pelliculage (exemple: dérivés cellulosiques, résines méthacryliques, chlorure de polyvinyle, nylons ...). Le produit vendu sous la marque de commerce Avicel est un produit de type cellulose microcristalline, fabriqué par FMC Corporation. Le produit vendu sous la marque de commerce Emcompress est un produit de type phosphate calcique, fabriqué par Edward Mendell Co. Inc.

Les compositions pharmaceutiques selon la présente invention se présentent généralement sous une forme solide. Il est important de souligner que les effets bénéfiques de l'invention sont observés, quelle que soit la présentation de la forme galénique. Les compositions pharmaceutiques selon la présente invention peuvent se présenter sous la forme de comprimés, de granules, de microgranules, etc., ces formes étant enrobées ou non.

Les compositions pharmaceutiques solides à libération contrôlée selon la présente invention peuvent être préparées par les diverses méthodes conventionnelles connues de l'homme du métier.

Généralement, les compositions pharmaceutiques solides selon la présente invention sont préparées par un procédé comprenant les étapes successives suivantes:
i. préparation d'un mélange contenant les composants a, b, c et d et les autres excipients éventuellement présents, éventuellement après granulation de certains composants et excipients; et
ii. la compression du mélange obtenu à l'étape i.

De préférence, on prépare un mélange homogène à l'étape i.

La compression peut être de différents types, et se fait idéalement par compression directe. La granulation éventuelle peut soit se faire par voie humide ou par voie sèche, ou encore par granulation par fusion (melt-granulation).

Selon un mode d'implémentation particulier de l'invention, les compositions pharmaceutiques solides à libération contrôlée selon l'invention peuvent être utilisées en combinaison avec une ou plusieurs compositions pharmaceutiques solides permettant la libération immédiate de substances actives. Lorsque ces deux types de compositions sont présentes dans une même entité, cela permet d'obtenir en une seule administration à la fois la libération immédiate d'une première substance active et la libération prolongée de la même ou d'une deuxième substance active.

C'est pourquoi la présente invention concerne également des compositions pharmaceutiques solides administrables par voie orale comprenant
A. au moins une couche comprenant une substance active et des excipients qui permettent la libération immédiate de ladite substance active après administration, et
B. au moins une deuxième couche qui permet la libération contrôlée de la même ou d'une deuxième substance active, comprenant ladite même ou deuxième substance active, au moins un excipient de type matriciel, au moins un polymère entérosoluble et au moins un agent alcalinisant.

En ce qui concerne la couche A, les excipients permettant la libération immédiate de la substance active peuvent être choisis parmi les diluants (emcompress, lactose ...), les liants (Avicel, amidons, polyvinylpyrrolidone ...), les désintégrants (amidons et amidons modifiés, dérivés cellulosiques, dérivés alginiques, pectines ...), les lubrifiants et agents d'écoulement (talc, stéarate de magnésium, silice colloïdale ...), les agents de masquage de goût (α-cyclodextrine, β-cyclodextrine, γ-cyclodextrine et leurs dérivés), des arômes ou des colorants.

De telles compositions pharmaceutiques solides combinées peuvent être préparées selon diverses méthodes connues de l'homme du métier.

Plus particulièrement, ces compositions pharmaceutiques solides combinées peuvent se présenter sous la forme d'un comprimé dans lequel au moins une couche A est accolée à au moins une couche B. Dans ce cas, de telles compositions pharmaceutiques solides peuvent être préparées par un procédé comprenant les étapes successives suivantes:
1) préparation de mélanges homogènes séparés à partir des composants des couches A et B, et
2) compression des mélanges homogènes obtenus en 1) dans une comprimeuse multicouches.

Eventuellement, l'étape de compression 2) peut être précédée par une étape de granulation de certains composants.

Les comprimeuses multicouches permettant de préparer ce genre de comprimés sont les comprimeuses multicouches de type Courtoy, Manesty, Hata, Fette, Killian, ...

Les comprimés multicouches sont particulièrement bien adaptés aux cas des associations de substances actives pour lesquelles des effets thérapeutiques bénéfiques bien particuliers ont été récemment observés, par exemple, pseudoéphédrine /cétirizine, hydrocodone/acétaminophen, hydrocodone libération immédiate/hydrocodone libération prolongée.

L'avantage qu'apporte cette nouvelle composition est d'autant plus surprenant que jusqu'à présent, les polymères entérosolubles étaient essentiellement utilisés pour effectuer le pelliculage (coating) de comprimés. En effet, un ouvrage de référence tel que le Handbook of Pharmaceutical Excipients (A. Wade and P. J. Weller - American Pharmaceutical Association - 1994 ) ne mentionne les polymères entérosolubles que pour leur utilisation comme agent de pelliculage.

Un autre avantage est de pouvoir limiter l'administration de substances actives à un comprimé par jour. Ceci étant possible grâce à la libération régulière de la substance active tout au long du tractus gastro-intestinal que permet cette nouvelle composition.

L'invention peut aussi avantageusement s'appliquer aux substances actives moins bien absorbées dans la partie basse du tractus intestinal en assurant une libération importante au niveau des premières portions de l'intestin et ainsi favoriser son absorption.

L'invention peut également avantageusement s'appliquer aux substances actives basiques.

L'invention est particulièrement adaptée aux substances actives dont la base libre est moins soluble dans l'eau que ses sels pharmaceutiquement acceptables.

Les exemples qui suivent illustrent la présente invention, sans toutefois la limiter. Dans les exemples qui suivent, les pourcentages sont exprimés en poids par rapport au poids total des compositions.

### Exemple 1. Utilisation d'un polymère entérosoluble acrylique et de carbonate de sodium comme agent alcalinisant.

Des comprimés de pseudoéphédrine dosés à 240 mg ont été préparés à partir des mélanges dont les compositions sont reprises dans le tableau 1.

**Tableau 1. -**

| Composition des comprimés A, B et C. | | | |
|---|---|---|---|
| composants | mg/comprimé | | |
| | A | B | C |
| Pseudoéphédrine.HCl | 240 | 240 | 240 |
| Carbonate de sodium | 97,5 | 97,5 | 0 |
| Natrosol 250 HHX™ | 110 | 110 | 110 |
| Eudragit L100-55™ | 0 | 55 | 55 |
| Avicel PH 102™ | 34,8 | 39,8 | 39,8 |
| Aérosil 200™ | 2,7 | 2,7 | 2,7 |
| Stéarate de magnésium | 5 | 5 | 5 |

Le Natrosol 250 HHX est une marque de commerce pour un produit de type hydroxyéthyl cellulose. L'Avicel PH 102 est une marque de commerce pour un produit de type cellulose microcristalline. L'Aérosil 200 est une marque de commerce pour un produit de type dioxyde de silicium colloïdal.

Les cinétiques de libération in vitro de la pseudoéphédrine à partir de ces trois types de comprimés ont été déterminées in vitro à l'aide de l'appareil de dissolution n° 1 de l'USP 23 (23ème édition de la pharmacopée américaine). Les comprimés sont placés dans le panier qui subit 100 rotations par minute. Les milieux de dissolution sont constitués de 900 ml de solution 0,1 N d'acide chlorhydrique ou de tampon phosphate à pH 7,5 maintenus à 37°C. Des prélèvements sont effectués après 1 h, 2 h, 4 h, 8 h, 12 h, 16 h et 20 h et la pseudoéphédrine est dosée par HPLC. Les résultats des dosages sont repris dans le tableau 2.

**Tableau 2. -**

| Pourcentages de libération de la pseudoéphédrine dans les comprimés A, B et C | | | | | | |
|---|---|---|---|---|---|---|
| Temps (h) | A | | B | | C | |
| | pH 1,1 | pH 7,5 | pH 1,1 | pH 7,5 | pH 1,1 | pH 7,5 |
| 0 | 0 | 0 | 0 | 0 | 0 | 0 |
| 1 | 17,0 | 13,2 | 12,5 | 18,0 | 39,1 | 57,1 |
| 2 | 29,0 | 22,0 | 20,6 | 29,4 | 57,6 | 78,9 |
| 4 | 46,0 | 36,7 | 36,2 | 47,0 | 79,8 | 95,0 |
| 8 | 69,7 | 55,6 | 63,7 | 70,7 | 96,9 | 102,3 |
| 12 | 86,3 | 67,7 | 82,7 | 86,3 | 99,6 | 103,6 |
| 16 | 96,2 | 76,8 | 94,6 | 96,8 | 99,5 | 104,4 |
| 20 | 100,3 | 83,7 | - | - | - | - |

Les résultats du tableau 2 montrent que le comprimé A, qui contient un alcalinisant mais pas d'agent entérosoluble, fournit des courbes de dissolution assez différentes selon le pH du milieu. La différence de surface sous la courbe est d'environ 25 %. Si on ajoute au comprimé un polymère entérosoluble (comprimés B), cette différence est très fortement réduite (différence de surface sous la courbe inférieure à 10%). On peut aussi noter qu'en absence d'agent alcalinisant (comprimés C), la libération est rapide.

Ce qui est particulièrement inattendu, est l'action régulatrice de l'agent alcalinisant en milieu basique. En effet, contrairement à toute attente, celui-ci réduit la vitesse de libération du composé actif et semble ralentir la solubilisation du polymère entérosoluble (Tableau 2).

De même, en milieu acide, rien ne laissait présager que l'agent alcalinisant n'entraîne aucune dissolution du polymère entérosoluble. Au contraire, le tableau 2 montre qu'à pH acide, l'agent alcalinisant et le polymère entérosoluble combinent leurs actions.

### Exemple 2. Utilisation de polymères entérosoluble cellulosiques et de carbonate de sodium comme agent alcalinisant.

Des comprimés de pseudoéphédrine dosés à 240 mg ont été préparés à partir des mélanges dont les compositions sont reprises dans le tableau 3.

**Tableau 3. -**

| Composition des comprimés D, E et F. | | | |
|---|---|---|---|
| composants | mg/comprimé | | |
| | D | E | F |
| Pseudoéphédrine | 240 | 240 | 240 |
| Carbonate de sodium | 97,5 | 97,5 | 97.5 |
| Natrosol 250 HHX™ | 110 | 110 | 110 |
| Aqoat AS-LF™ | 55 | 0 | 0 |
| Aqoat AS-MG™ | 0 | 55 | 0 |
| Aqoat AS-HF™ | 0 | 0 | 55 |
| Avicel PH 102™ | 39,8 | 39,8 | 39,8 |
| Aérosil 200™ | 2,7 | 2,7 | 2,7 |
| Stéarate de magnésium | 5 | 5 | 5 |

L'Aqoat AS-LF, l'Aqoat AS-MG et l'Aqoat AS-HF sont des marques de commerce pour un produit de type hydroxypropylméthylcellulose acétate succinate.

Les cinétiques de libération in vitro de la pseudoéphédrine à partir de ces trois types de comprimés ont été déterminées in vitro à l'aide de l'appareil de dissolution n° 1 de l'USP 23 (23ème édition de la pharmacopée américaine). Les comprimés sont placés dans le panier qui subit 100 rotations par minute. Les milieux de dissolution sont constitués de 900 ml de solution 0,1 N d'acide chlorhydrique ou de tampon phosphate à pH 7,5 maintenus à 37°C. Des prélèvements sont effectués après 1 h, 2 h, 4 h, 8 h, 12 h et 16 h et la pseudoéphédrine est dosée par HPLC. Les résultats des dosages sont repris dans le tableau 4.

**Tableau 4. -**

| Pourcentages de libération de la pseudoéphédrine dans les comprimés D, E et F | | | | | | |
|---|---|---|---|---|---|---|
| Temps (h) | D | | E | | F | |
| | pH 1,1 | pH 7,5 | pH 1,1 | pH 7,5 | pH 1,1 | pH 7,5 |
| 0 | 0 | 0 | 0 | 0 | 0 | 0 |
| 1 | 13,6 | 18,5 | 13,2 | 14,4 | 15,0 | 16,5 |
| 2 | 23,4 | 29,4 | 21,1 | 28,3 | 23,6 | 26,8 |
| 4 | 41,2 | 46,0 | 34,7 | 40,3 | 38,0 | 43,8 |
| 8 | 67,4 | 67,1 | 56,9 | 63,2 | 60,6 | 66,5 |
| 12 | 86,1 | 81,4 | 74,6 | 79,1 | 78,2 | 80,9 |
| 16 | 99,6 | 90,7 | 87,8 | 90,4 | 92,5 | 92,4 |

Les résultats du tableau 4 montrent que pour les 3 types de comprimés les différences de cinétique de libération sont très faibles entre les 2 pH étudiés. La différence de surface sous la courbe est d'environ 1% pour D, 8,4% pour E et 6,1% pour F.

## Revendications

1. Composition pharmaceutique solide administrable par voie orale, permettant la libération contrôlée d'au moins une substance active, comprenant
a ladite au moins une substance active,
b entre 5 et 95 % en poids, par rapport au poids total de la composition, d'au moins un excipient matriciel, sélectionné parmi les matrices inertes, les matrices hydrophiles, les matrices lipidiques, les mélanges de matrices inertes et de matrices lipidiques, les mélanges de matrices hydrophiles et de matrices lipidiques, les mélanges de matrices hydrophiles et de matrices inertes;
c entre 2 et 50 % en poids, par rapport au poids total de la composition d'au moins un polymère entérosoluble ; et
d entre 0,5 et 50 % en poids, par rapport au poids total de la composition d'au moins un agent alcalinisant soluble dans une phase aqueuse dans des conditions de pH physiologique.

2. Composition pharmaceutique selon la revendication 1 , caractérisée en ce que la substance active est choisie parmi la pseudoéphédrine, l'éphédrine, la phényléphrine, la phénylpropanolamine, le trapidil, l'hydrocodone, la cétirizine, l'éflétirizine, l'hydroxyzine, la méclozine, la buclizine, la pentoxyvérine, la codéine, la morphine, la théophylline, le lévétiracetam, le piracétam, le valpromide, le valproate de sodium, la gabapentine, la carbamazépine, leurs isomères optiques ou leurs sels pharmaceutiquement acceptables.

3. Composition pharmaceutique selon l'une quelconque des revendications 1 à 2, caractérisée en ce que le polymère entérosoluble porte au moins un groupement acide.

4. Composition pharmaceutique selon l'une quelconque des revendications 1 à 3, caractérisée en ce que le polymère entérosoluble est choisi parmi les polymères cellulosiques ou les polymères acryliques.

5. Composition pharmaceutique selon la revendication 4, caractérisée en ce que le polymère entérosoluble est choisi parmi le groupe constitué de l'hydroxypropylméthylcellulose acétate succinate, les composés vendus sous la marque Aqoat, l'acétylphtalate de cellulose, le phtalate d'hydroxypropylméthylcellulose ou la cellulose acétate trimellitate, les copolyméres de l'acide méthacrylique, tels que les composés vendus sous la marque Eudragit.

6. Composition pharmaceutique selon l'une quelconque des revendications 1 à 2, caractérisée en ce que l'agent alcalinisant soit choisi parmi les hydroxydes, les carbonates, les bicarbonates, les phosphates de métaux alcalins ou alcalino-terreux, le borate de sodium ainsi que des sels basiques d'acides organiques.

7. Composition pharmaceutique selon l'une quelconque des revendications 1 à 6, caractérisée en ce qu'elle comprend en outre un ou plusieurs autres excipients pharmaceutiquement acceptables.

8. Procédé de préparation d'une composition pharmaceutique solide selon l'une quelconque des revendications 1 à 7, caractérisé en ce qu'il comprend les étapes successives suivantes:
i. préparation d'un mélange contenant les composants a, b, c et d et les autres excipients éventuellement présents, éventuellement après granulation des composés et des excipients; et
ii. la compression du mélange obtenu à l'étape i.
